# EUROPEAN PATENT SPECIFICATION

(11) **EP 0 531 407 B1**
(45) Date of publication and mention of the grant of the patent: **09.02.2000**
(21) Application number: 91910478.6
(22) Date of filing: 07.05.1991
(51) Int. Cl.: C12N 15/16, A61K 38/24, A61K 38/08, C07K 14/00

(54) **ANALOGS OF GLYCOPROTEIN HORMONES HAVING ALTERED IMMUNOLOGICAL CHARACTERISTICS, EFFICACY AND/OR RECEPTOR SPECIFICITY**
GLYKOPROTEINHORMON-ANALOGA MIT VERÄNDERTEN IMMUNOLOGISCHEN MERKMALEN, VERÄNDERTER WIRKSAMKEIT UND/ODER REZEPTORSPEZIFITÄT
ANALOGUES D'HORMONES GLYCOPROTEIQUES AYANT DES CARACTERISTIQUES IMMUNOLOGIQUES, UNE EFFICACITE ET/OU UNE SPECIFICITE DE RECEPTION MODIFIEES

(30) Priority: 08.05.1990 US 520703
(43) Date of publication of application: 17.03.1993
(73) Proprietor: UNIVERSITY OF MEDICINE & DENTISTRY OF NEW JERSEY, Newark, New Jersey 07107-3000 (US)
(72) Inventor: CAMPBELL, Robert, K., Pennington, NJ 08854 (US); MOYLE, William, R., Piscataway, NJ 08854 (US)
(74) Representative: VOSSIUS & PARTNER
(86) International application number: US9103162
(87) International publication number: WO9116922

(56) References cited:
- The Journal of Cell Biology, Volume 109, issued October 1989, M.M. MATZUK et al., "Mutagenesis and Chimeric Genes Define Determinants in the beta subunits of Human Chorionic Gonadotropin and Lutropin for Secretion and Assembly", pages 1429-1438, see especially Abstract and page 1434, col. 1.
- Proceedings National Academy of Science, USA, Volume 88, issued February 1991, R.K. CAMPBELL et al., "Conversion of Human Choriogonadotropin into a follitropin by protein engineering", pages 760-764, see entire document.
- Proceedings National Academy of Science, USA, Volume 88, issued February 1991, R.K. CAMPBELL et al., "Conversion of Human Choriogonadotropin into a follitropin by protein engineering", pages 760-764, see entire document.

## Description

The invention relates to glycoprotein hormone analogs and other proteins, their methods of use and other applications. The molecules of the invention are analogs or derivatives of naturally occurring vertebrate glycoprotein hormones and DNA molecules encoding them. The naturally occurring hormones are typically constituted of an alpha and beta subunit. Amino acid sequences of naturally occurring alpha and beta-subunits from several different species are presented in TABLE I. Monomeric forms of both subunits as well as heterodimers and homodimers are known to occur. In the molecules of the invention the chemical structure of the beta-subunit or the alpha-subunit or both may be different from that of the native hormones. The glycoprotein hormone analogs of the invention also differ from the naturally occurring hormones in their biological, physiological, pharmaceutical and/or immunological characteristics. Observations that many different changes can be made in the subunits of naturally occurring glycoprotein hormones which can selectively alter specific properties of the molecules has led to increased knowledge of hormone structure. The hormone derivatives of the invention are useful as tools with which various endocrine and metabolic functions can be manipulated and dysfunctions can be treated. One aspect of the invention relates to the control of reproduction in mammals.

The hormones of the invention are useful in numerous applications including therapeutic, immunological, diagnostic, and other uses. More particularly, the hormones of the invention are capable of altering the reproductive process in living organisms such as mammals and other vertebrates, in particular the hormones of the invention promote fertility. The various aspects of the invention are discussed in full detail hereinafter. The hormones of the invention may be produced by genetic engineering, chemical synthesis and/or a combination of such methods. The glycoprotein hormones of the invention contribute to fulfilling an important and actual need in the relevant field of art.

The glycoprotein hormones include the gonadotropins and thyrotropins. In humans these are chorionic gonadotropin (CG), luteinizing hormone (LH), follicle stimulating hormone (FSH) and thyroid stimulating hormone (TSH). They are a family of heterodimeric proteins which share a common alpha-subunit but differ in their hormone-specific beta-subunits. Gonadotropins such as CG, LH and FSH play a major role in the reproductive process, while TSH is important for thyroid function. Owing to the importance of these hormones in endocrine and metabolic function, there has been considerable interest in characterizing the relationship between hormone structure and function.

While primary structures of many glycoprotein hormones have been known for many years, the relationship between hormone structure and function has been only partially characterized in spite of many studies involving chemical modifications (reviewed in 1), deglycosylation (1-4), immunological characterization (5-8), and use of peptide analogs (9-12). The beta-subunit specifies the activity of the hormones, however, neither free subunit has much biological activity (1). The reason the alpha, beta-heterodimer is the most active form is not known. Its activity might be the result of a unique conformation of one or both subunits which forms after they combine (13) and/or it might be that portions of both subunits interact directly with the receptor (9, 10). The observation that some antibodies bind to epitopes on the hCG beta-subunit only when it is associated with the alpha-subunit suggests that the conformation of the beta-subunit is altered when it combines with the alpha-subunit (14). Residues responsible for the binding of these antibodies have been identified and include those between Cys-38 and Cys-57 (15), a region of the molecule which has been proposed to interact with the receptor (10). Similarly, some monoclonal antibodies and polyclonal antisera to the alpha-subunit (16) have higher affinity for the alpha, beta-heterodimer than for the free alpha-subunit, suggesting that the conformation of the alpha-subunit is altered when it combines with the beta-subunit. Evidence that both subunits participate in binding to the receptor is based on the ability of synthetic alpha and beta-subunit peptides to inhibit hCG binding to receptors (9, 10) and on the abilities of both subunits to be cross-linked to the receptor (17-19). It appears that the alpha-subunit may have a different conformation in different hormones since anti-alpha-subunit antibodies have been found which bind to the alpha-subunit in the heterodimeric forms of some but not all human glycoprotein hormones (29).

The Asn-linked carbohydrate residues influence efficacy of the hormones. Deglycosylation by chemical (3, 4), enzymatic (20, 21), or genetic (22) procedures renders them less active in vitro. The reasons the carbohydrate residues are required, particularly those on the alpha-subunit at Asn-52 (22), are unknown. The carbohydrate residues may participate in cross-linking the hormone-receptor complex to other cell surface proteins (2, 23). They may also alter the conformation of the molecules (24) although this has been hard to demonstrate spectroscopically (25). The carbohydrates may increase the flexibility of the hormones and facilitate a change in conformation which occurs after the hormones bind to the receptor (26). A report that Fab fragments of antibodies to hCG can convert the deglycosylated hormone into an agonist (27) lends further support for the effects of the carbohydrates on the conformation of hCG. Asn-linked sugars also appear to have a role in dimer stability since it is more difficult to dissociate the dimer following deglycosylation (28) and since deglycosylated subunits recombine faster than native alpha and beta-subunits (25).

There are two major barriers to the understanding of the relationship between glycoprotein hormone structure and function. First, the tertiary structures of the hormones have not been determined. The hormones resisted crystallization and only recently have high quality crystals for x-ray diffraction studies of a glycoprotein hormone (hCG) been obtained (71, 72). In addition, there is doubt regarding the locations of the disulfide bonds in the subunits (1, 2). Due to the difficulties anticipated in obtaining the tertiary structure of the carbohydrate chain, known to influence the activity of the hormone, even crystallographic and/or NMR approaches may not guarantee the characterization of residues which interact with hormone receptors to induce a response.

The second major impediment to understanding the relationship between structure and function has been the difficulty of preparing analogs. Until 1985 (30), the only means of preparing analogs was by chemical or enzymatic means. The limited numbers of residues which could be specifically modified and the difficulty of removing particular N-linked sugar chains curtailed progress substantially.

Present day recombinant DNA technology permits the production of a much wider range of analogs. Several glycoprotein hormones have now been expressed from DNA introduced into mammalian cells in culture, and analogs of some have also been expressed (22, 30-36).

The present invention utilizes DNA mutagenesis and gene expression to produce glycoprotein hormone analogs. These analogs have enabled identification of residues important for receptor binding and specificity, subunit interactions, and antibody binding.

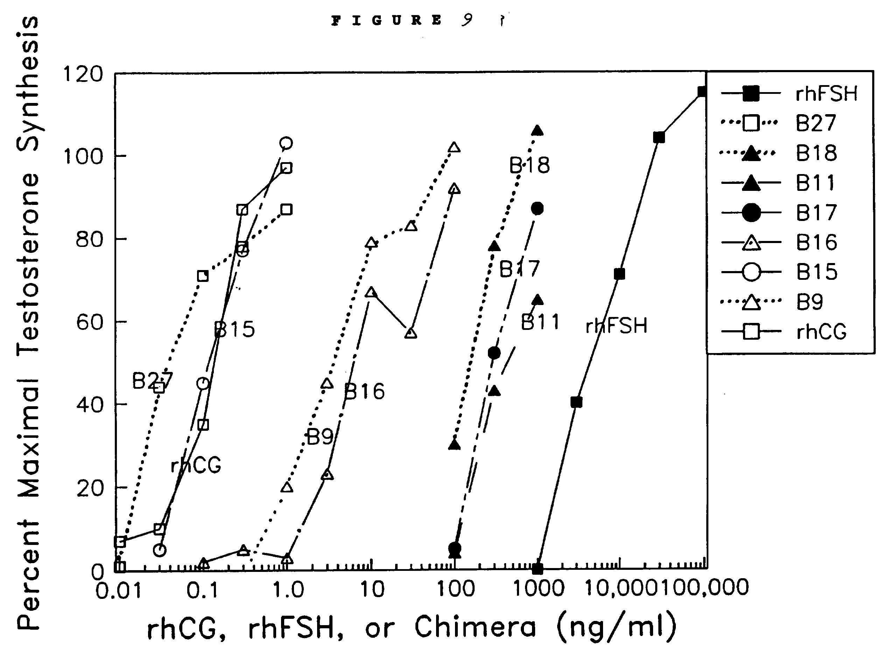

It has been discovered that a wide variety of changes can be made in these hormones. For example, chimeric subunits can be created which combine specific characteristics of each parent molecule such as receptor or antibody binding (TABLES II, III and FIGURE 9).

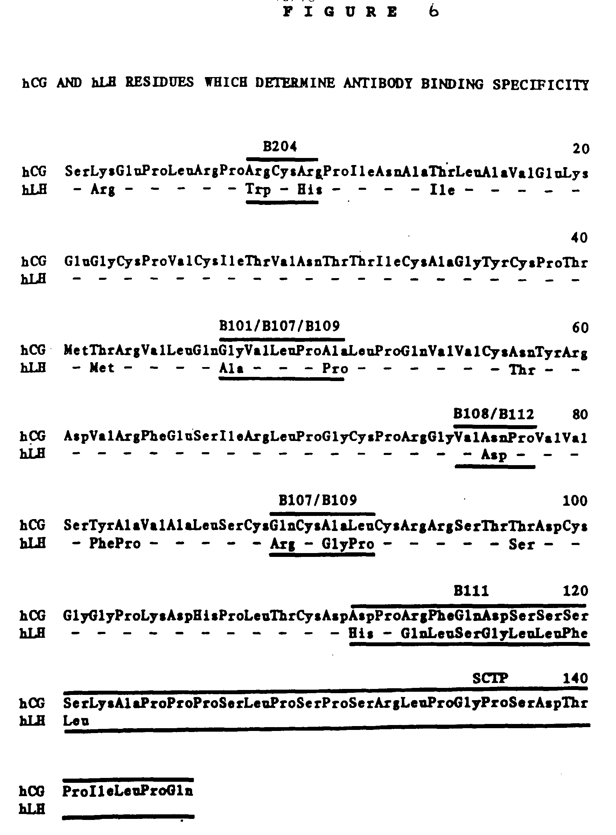

Identification of amino acid residues in the hormones that contribute to their biological, physiological, pharmacological, and/or immunological characteristics (FIGURES 6-10) has enabled the production of a variety of useful analogs. The invention provides for compounds useful for manipulation of gonad function. Among these uses are the induction of fertility in male and female mammals.

The glycoprotein hormones have been studied extensively. There has been considerable interest in the structure/function relationships of these molecules. The general references listed below reflect the current state of knowledge in this area.

PIERCE, J.G. and PARSONS. T.F., Ann. Rev. Biochem. 50, 465 (1981). This extensive review article summarizes the results of various chemically and enzymatically derived analogs of the naturally occurring glycoprotein hormones to discern the relationship between their structure and function. The authors conclude that much remains to be known about the interaction of the glycoprotein hormones with their receptors.

RYAN, R.J. et al., Recent Progress Hormone Res. 43, 383 (1987). RYAN, R.J. et al., "The Glycoprotein Hormones: Recent Studies of Structure-Function Relationships", FASEB Journal 2 2661 (1988). These articles summarize studies employing synthetic peptides to study glycoprotein hormone structure and function.

KEUTMANN, H.T. et al., Proc. Nat'l. Acad. Sci. USA 84, 2038 (1987). This work concludes that the intercysteine 38-57 loop sequence in the beta-subunit of hCG and hLH is a determinant for expression of biological activity in these molecules.

SCHNEYER, A.L. et al., Biochemistry 27, 666 (1988). This work concludes that hFSH beta-subunit sequences 33-52 are important in receptor binding, and that the sequence TRDL (34-37) is of particular importance.

An example of work identifying the role of the subunits in determining binding specificity is Strickland, T.W. and Puett,D., "Contribution of Subunits to the Function of Luteinizing Hormone/Human Chorionic Gonadotropin Recombinants", Endocrinology 109, 1933 (1981). Hybrid recombinants prepared from bovine and porcine LH and human CG were used. It was observed that both the alpha and beta-subunits were found to contribute to the properties of the recombinant in several in vitro systems. In each case, the recombinant behaved most like the hormone from which the beta-subunit was derived.

For a general reference on reproductive endocrinology, see Yen, S.S.C. and Jaffe, R.B., "Reproductive Endocrinology: Physiology, Pathophysiology and Clinical Management", 2nd edition, W.B. Saunders Company, Philadelphia (1986), hereinafter referred to as "Yen and Jaffe". Chapter 19 of this text reviews both the mechanisms involved in fertilization and the clinical problems of infertility. This text illustrates uses of glycoprotein hormones for induction of fertility.
FIGURE 1 depicts the nucleotide sequence and encoded amino acid sequence of the hCG beta-subunit cDNA along with several restriction endonuclease recognition sites.
FIGURE 2 depicts the nucleotide sequence and encoded amino acid sequence of the hCG alpha-subunit cDNA along with several restriction endonuclease recognition sites.
FIGURE 3 depicts the sequences DNA oligonucleotides used to construct plasmids pKBMt and pKBM.
FIGURE 4A depicts the nucleotide sequence and encoded amino acid sequence of the analog hCG-beta, which contains two silent mutations that eliminate restriction endonuclease recognition sites present in the hCG beta-subunit cDNA; and FIGURE4B depicts the DNA oligonucleotides used to construct this analog by cassette mutagenesis.
FIGURE 5 depicts the nucleotide sequences of DNA oligonucleotide cassettes used to construct analogs B9 and B11 (see TABLE II).
FIGURE 6 illustrates hCG beta-subunit residues critical for antibody binding.
FIGURE 7 is a dose response curve of LH receptor binding by hCG/hFSH chimeric beta-subunits co-expressed in COS7 cells with human alpha-subunit, compared with hCG and hFSH.
FIGURE 8 is a dose response curve of FSH receptor binding by hCG/hFSH chimeric beta-subunits co-expressed in COS7 cells with human alpha-subunit, compared with hCG and hFSH.
FIGURE 9 is a dose response curve of LH receptor mediated steroidogenic activity of hCG/hFSH chimeric beta-subunits co-expressed in COS7 cells with human alpha-subunit, compared with hCG and hFSH.
FIGURE 10 is a dose response curve of FSH receptor mediated steroidogenic activity of hCG/hFSH chimeric beta-subunits co-expressed in COS7 cells with human alpha-subunit, compared with hCG and hFSH.

TABLE I depicts amino acid sequences of several naturally occurring glycoprotein hormone alpha-subunits and beta-subunits.

TABLE II depicts amino acid sequences of several hCG/hFSH beta-subunit chimeras.

TABLE III illustrates monoclonal antibody binding to chimeric hFSH/hCG beta-subunits.

The invention provides protein hormone analogs which are "engineered" such that they are capable of controlling various hormone-regulated physiological functions in vertebrates of both genders. In particular, the invention relates to the glycoprotein hormones and their individual subunits. The heterodimeric forms of the hormones are thought to be the most active. The four human hormones belonging to this group are chorionic gonadotropin (CG), luteinizing hormone (LH),follicle stimulating hormone (FSH) and thyroid stimulating hormone (TSH).

In accordance with the invention, the terminology "hormone analogs", "glycoprotein hormone analogs" or "variants" is to be understood in the broadest manner. Within the description the term "chimera" is used to designate a hormone analog which contains amino acid sequences derived from two or more different glycoprotein hormones. These chimeras can be constructed from proteins with low sequence homology. For example, beta-subunit chimeras can be made from hCG and hFSH beta-subunits in regions of very low sequence homology (TABLE II). These chimeras, described in detail below, exhibit useful properties such as the ability to bind and stimulate both LH and FSH receptors. In some instances comparatively small changes, such as modification of a single amino acid in the hormone sequence, cause significant alteration of the activity as compared to the native hormone.

The analogs contemplated by the invention include molecules which have novel biological, physiological, pharmacological and/or immunological (hereinafter "biological") activities and molecules that are useful intermediates for such active molecules. Within the scope of the invention are modified coupled dimers. Within the scope of the invention are applications and uses in the human field, in the veterinary field, and other fields of use, including applications in other vertebrates. As described, the analogs of the invention have the property of influencing fertility (e.g., promoting) in humans and animals.

In accordance with the invention, particular domains of amino acids are identified to which specific physiological functions or other biological activities can be attributed (FIGURES 6-10). The invention encompasses variants of the beta-subunit which in the dimer or monomer form exhibit new or altered biological activities. It is contemplated that the analogs of the invention will be useful as agonists.

The invention provides beta-subunit chimeras that are capable of directing hormone binding to both LH and FSH receptors. These chimeras may be derived from human glycoprotein hormones (e.g., hCG/hFSH or hLH/hFSH) or non-human glycoprotein hormones (e.g., bovine LH/bovine FSH or equine LH (or CG)/equineFSH), however, only the hCG/hFSH chimeras as mentioned below are comprised by the present invention. It is contemplated that these chimeras will be useful for the treatment of infertility in men and women and the promotion of fertility in male and female animals.

The invention includes analogs of glycoprotein hormones capable of the induction of fertility in men, women and animals of both genders.

### INDUCTION OF FERTILITY IN WOMEN, MEN AND ANIMALS

Infertility is a significant clinical, social and human health problem. Many women who desire to become pregnant fail to develop a dominant follicle and trigger the hCG surge needed for ovulation. If an ovulation is caused by gonadotropin deficiency, such as in polycystic ovarian disease, follicular maturation and ovulation can be induced by administration of human menopausal gonadotropin (hMG) followed by hCG. hMG is an extract prepared from the urine of post-menopausal women containing hLH and hFSH. It is sold commercially under the tradename Perganol(TM).

In women both hLH and hFSH are necessary for ovarian steroidogenesis, follicle maturation, and ovulation. During follicular development in the ovary, LH stimulates the cells surrounding the follicle and induces androgen production. Cells inside the follicle surrounding the oocyte are responsive to FSH and convert the androgens produced by the thecal and interstitial cells into estrogens.

Certain constructs of the invention are contemplated to be useful for the treatment of female infertility caused by gonadotropin deficiency. These molecules interact with LH and FSH receptors such that when administered to an infertile female, estrogen formation and follicular maturation should be induced. Thus, the invention discloses a protein which can replace Perganol (TM), hMG or other mixtures of hLH and hFSH in the treatment of infertility.

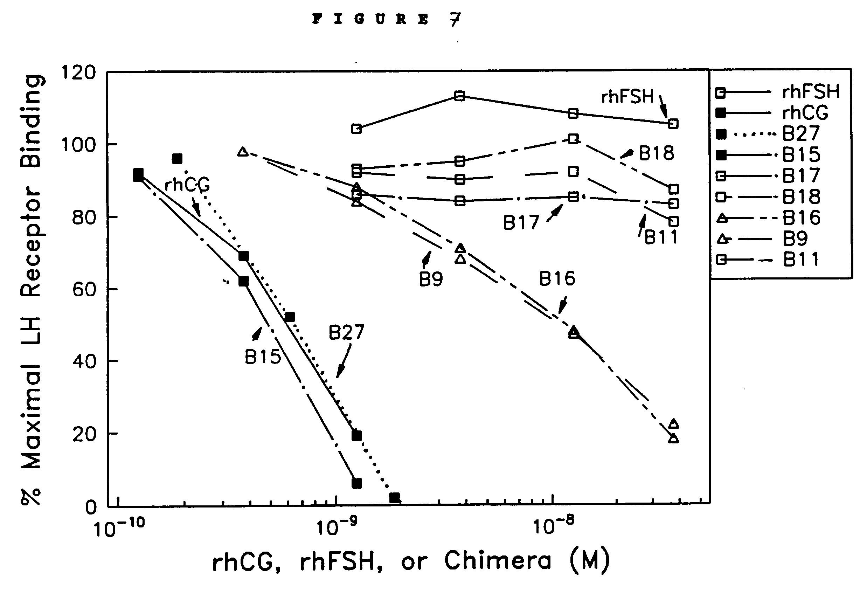

Preferred embodiments of the invention are chimeras in which residues near the carboxy-terminal region of hFSH are substituted for the corresponding region of hCG. These chimeras combine with the human alpha-subunit to form a dimer which has biological activity. In the following, for a better understanding of the present invention, four specific chimeras are discussed. These chimeras are not included within the scope of the present invention. The sequences of these constructs are shown in TABLE II and are denoted B11, B17, B18 and B19. All of these constructs combine with the alpha-subunit to form a heterodimer which can bind LH receptors and stimulate Leydig cell steroidogenesis (20) as well as bind FSH receptors and stimulate granulosa cell steroidogenesis (37). Antibody binding to these analogs has also been characterized. Results of representative assays are indicated in TABLE III and FIGURES 7-10.

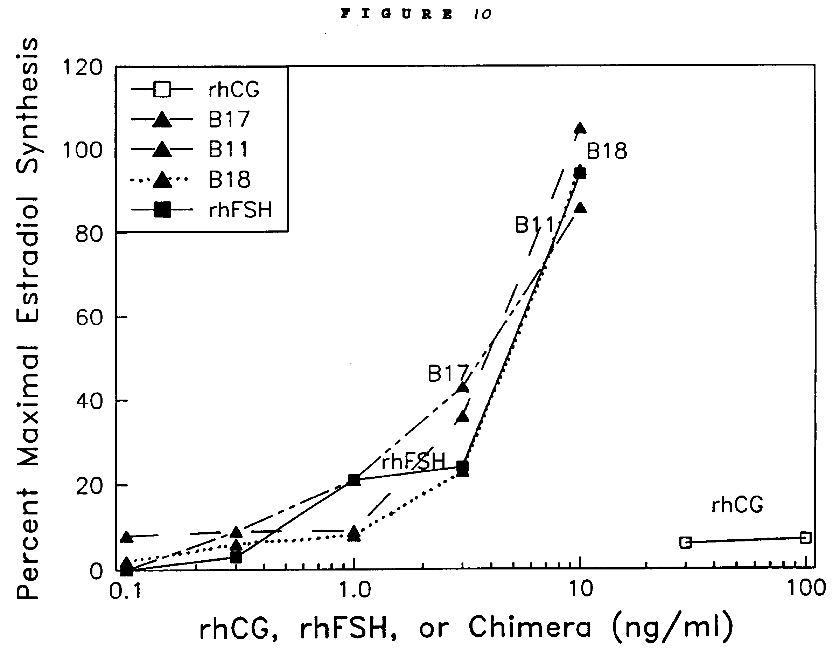

One of the chimeras is construct B18. This construct is alternately designated beta-CF94-114, where C and F indicate the pattern of hCG-beta and hFSH-beta sequences, respectively, and the numbers indicate the first and last non-identical residues in each region of substitution. Construct B18 is also alternately designated DG'. In this construct, residues 88-108 of hFSH-beta replace residues 94-145 of hCG-beta. This construct combines with the alpha-subunit to form a heterodimer that can bind both LH and FSH receptors and stimulate steroidogenesis (FIGURES 9 and 10).

Other embodiments of the hCG/hFSH beta-subunit chimeras are constructs B11, B17, and B19. Construct B11, alternately designated beta-CF94-117 or DG, contains residues 88-111 of hFSH-beta in place of residues 94-145 of hCG-beta. Construct B17, alternately designated beta-CFCF39-58/94-117 or ABCDG, contains residues 33-52 and 88-111 of hFSH-beta in place of residues 39-58 and 94-145 of hCG-beta, respectively. Construct B19, alternately designated beta-CFCF39-58/94-113, 114Q or ABCDG', is a variant of construct B17 which combines chimeric and non-chimeric substitutions and a deletion. The amino acid sequence is the same as B17 except that a glutamine (Q) is substituted for glutamic acid (E) at position 114, and the last three residues (115-117) are deleted.

The embodiments which are comprised by the present invention are chimeras in which smaller regions of of hFSH-beta such as the sequence DSDSTDCTVRGLGPSY (hFSH-beta residues 88-103), TVRGLGPSY (hFSH-beta residues 95-103) or TVRGLG (hFSH-beta residues 95-100) are inserted into appropriate regions of the hCG beta-subunit. The resultant chimeric beta-subunit directs binding of the alpha, beta-heterodimer to FSH receptors, eliciting a biological response in addition to the molecule's native LH receptor binding activity and efficacy. Although the above smaller regions of hFSH beta may also be inserted into appropriate regions of the hLH beta-subunit to result in chimeric beta-subunits with the above indicated activity, such chimeric subunits are not comprised by the present invention.

Further constructs which, however, are not included in the present invention are those chimeras in which the hCG-beta or hLH-beta sequences can be substituted at the carboxy terminal of hFSH, as for construct B25, and smaller regions of hCG-beta can be substituted into hFSH-beta, as for construct B26, whereby these chimeric proteins would direct binding of the alpha, beta-heterodimer to LH receptors, eliciting a biological response. It is thereby contemplated that these various chimeras may exhibit differing potencies of activity at the LH and FSH receptors, leading to the selection of one over the others for certain applications because of its higher or lower ratio of FSH to LH activity.

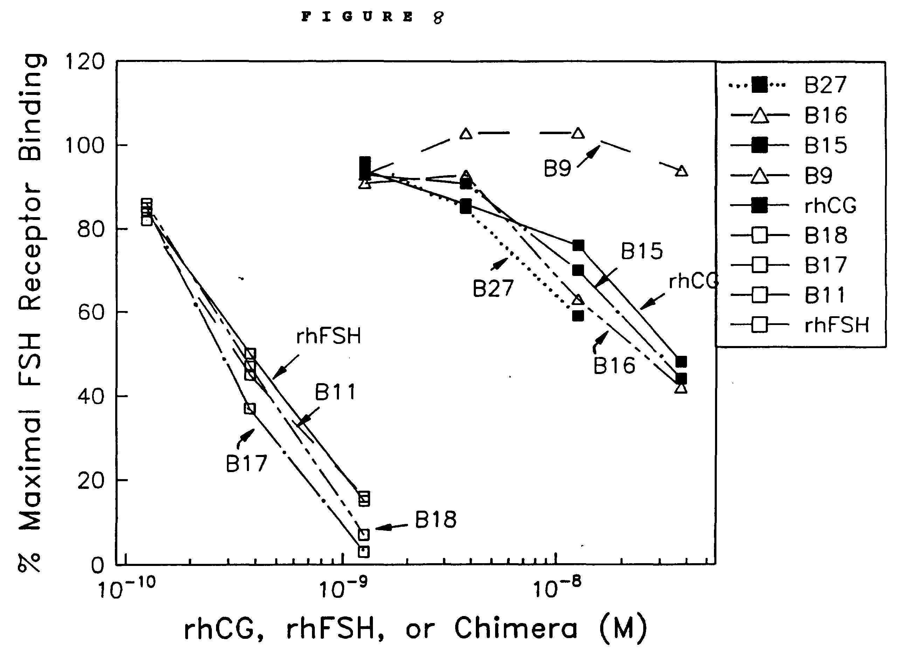

The finding that the constructs of the invention containing hFSH-beta residues in place of hCG-beta residues 94-145 will direct binding to LH and FSH receptors and stimulate steroidogenesis was an unexpected one. The prior art shows that other regions of the beta-subunit were thought to be important for receptor binding. Schneyer et al. (12) indicate that the TRDL sequence of hFSH-beta (residues 34-37) are necessary for binding to FSH receptors. Subsequent studies by Santa Coloma et. al. (76) indicate that hFSH-beta residues 33-53 bind and stimulate FSH receptors. Keutmann et al. (10) concluded that corresponding region of hCG-beta and hLH-beta, residues 38-57, were necessary for binding and stimulation of LH receptors. These authors also concluded that hCG-beta residues 93-101 were necessary for binding to LH receptors. If these regions of the beta-subunit were important for receptor binding specificity, chimeric hCG/hFSH beta-subunits containing sequence alterations in these regions would exhibit altered binding specificity. This is not the case. Data obtained with constructs B9, B15, and B16 reflect this (FIGURES 7-10). Construct B15, alternately designated CFC39-58 or ABC, contains hFSH-beta residues 33-52 in place of hCG-beta residues 39-59. The receptor binding activity of the alpha, beta-B15 heterodimer is virtually indistinguishable from hCG (FIGURES 7 and 8). Construct B9, alternately designated CFC94-97 or D, contains hFSH-beta residues 88-91 in place of hCG-beta 94-97 (residues corresponding to hCG-beta 98-100 are identical in hFSH-beta and hCG-beta). The alpha, beta-B9 heterodimer binds and stimulates LH receptors, but with about 30-fold less potency than hCG (FIGURES 7 and 9). The alpha, beta-B9 heterodimer exhibits no FSH receptor binding at the amounts tested (FIGURE 8). Construct B16, alternately designated CFCFC39-58/94-97 or ABCD, combines the substitutions present in B9 and B16. As for B9, the alpha, beta-B16 heterodimer is about 30-fold less active than hCG at LH receptors (FIGURES 7 and 9). It is no better than hCG in binding to FSH receptors (FIGURE 8).

It is further contemplated that the chimeric constructs of the invention and also those specific ones mentioned above, however, not included within the present invention would be useful for the enhancement of fertility in men and animals of male gender, i.e., to stimulate spermatogenesis. Induction of spermatogenesis requires both LH and FSH. LH stimulates testosterone production by Leydig cells of the testes. FSH and the high local concentrations of testosterone produced in response to LH stimulate the Sertoli cells and thereby induce spermatogenesis. For this reason, compounds that act on both FSH and LH receptors are desirable. Further, since it is known that hCG has a much longer half-life than FSH, it would be expected that hormone analogs containing hCG/hFSH-beta-subunit chimeras would have a longer biological half-life than hFSH. These analogs would be anticipated to be more efficacious than hCG or hFSH alone or mixtures of hCG and hFSH.

### OTHER CHIMERAS

The invention also relates to other glycoprotein hormone chimeras or analogs. It is contemplated that these chimeras will be useful for structure/function residue mapping and fertility-influencing compounds for various animal species.

Thus, the alpha and beta-subunit sequences in the chimeras can be from any vertebrate (e.g., mammal, bird, amphibian, reptile or fish). However, only alpha, beta-heterodimeric polypeptides with human CG and FSH sequences are comprised by the present invention. It is to be noted that notwithstanding the various structural changes discussed herein, the molecules of the invention fold into the proper conformation as is apparent from the fact that they have biological activity.

### METHODS OF USE

The compounds of the invention can be formulated into suitable pharmaceutical, physiologically active compositions. These compositions may be used to be administered intravenously, intramuscularly, parenterally, orally or by other routes appropriate for proteins. The amount of the active analog of the invention will be used in an amount sufficient to promote the desired physiological response but preferably not in an amount in excess of that necessary to cause an optimum response. The amount may vary from a few nanograms to about 10 kilograms, the amount being dependent of the human or animal to whom the administration is made, the condition to be treated and other variables to be taken into account. One skilled in the art will determine without undue experimentation the optimum amounts to be administered. The compositions will normally include a pharmaceutically or physiologically acceptable carrier or diluent with the active protein of the invention. The compositions may be in any suitable form, like injectable preparations.

### EXAMPLES

The following EXAMPLES are provided by way of illustration and not by way of limitation. One skilled in the art is capable without undue experimentation, to vary the components and thus construct other analogs. EXAMPLE 1 describes the insertion of hCG alpha-subunit and beta-subunit cDNAs into the eukaryotic expression vector pSVL. EXAMPLE 2 describes the construction of the vectors pKBM, pKBM-hCG-alpha and pKBM-hCG-beta. EXAMPLE 3 describes the construction of pKBM-hCG-beta', which contains two silent mutations in the hCG-beta cDNA nucleotide sequence resulting in the removal of two restriction sites. EXAMPLE 4 describes a chimeric hCG/hFSH beta-subunit, analog B9 (see TABLE II). EXAMPLE 5 describes the construction of a chimeric hCG/hFSH beta-subunit, analog B11 (see TABLE II). EXAMPLE 6 describes the construction of the expression vector pCM, which can be used to stably transfect COS-1 and COS-7 cells. EXAMPLE 7 describes the construction of pBMT2X-hCG-alpha and pBMT2X-hCG-beta expression constructs for use in C127 cells. EXAMPLE 8 describes techniques used in mutagenesis and preparation of analogs. EXAMPLE 9 describes transfection of mammalian cells(COS-1, COS-7, C127, CHO) with plasmid DNA. EXAMPLE 10 describes immunological assays of the analogs. EXAMPLE 11 describes assays of receptor binding. EXAMPLE 12 describes assays of receptor stimulation (steroidogenesis or cAMP accumulation).

Unless indicated otherwise, standard molecular biological techniques (e.g., ref. 41) were used. Restriction endonucleases and other DNA modification enzymes (e.g., T4 DNA polymerase, Thermus aquaticus (Taq) DNA polymerase, T4 DNA ligase, calf intestine alkaline phosphatase, bacterial alkaline phosphatase) were obtained from New England Biolabs, Inc., Bethesda Research Laboratories (Life Technologies), Inc. (BRL), Boehringer Mannheim, United States Biochemical Corp., or Cetus, and were used as suggested by the manufacturer unless indicated otherwise. Subcloning efficiency competent HB101 and DH5-alpha E.coli were obtained from BRL and transformed as suggested by the manufacturer with modifications as noted. DNA sequencing was performed using a Sequenase (TM) kit (United States Biochemical Corp.) as suggested by the manufacturer. COS (COS-1 or COS-7), C 127 and CHO-K1 (CHO) cells were obtained from the American Type Culture Collection (ATCC-Rockville, MD). COS-1, COS-7 and C127cells were routinely maintained in Dulbecco's modified Eagle's medium (DMEM) containing 10% fetal calf serum (FCS) or 10% supplemented calf serum. CHO cells were cultured in F-12 medium containing 10% FCS as suggested by ATCC. Tissue culture reagents were obtained from GIBCO Laboratories (Life Technologies) Inc. and HyClone Laboratories, Inc. Monoclonal antibodies were provided by Drs. R.E. Canfield (Columbia Univ., New York NY), R.J. Ryan (Mayo Clinic, Rochester, MN), E.G. Armstrong and R. Wolfert (Hybritech Corp., San Diego, CA), and R. Krogsrud and S.Berube (Biomega Diagnostics, Montreal, Canada).

### EXAMPLE 1

### Construction of pSVL-hCG-beta and pSVL-hCG-alpha

The cDNAs for the hCG-alpha and beta-subunits were cloned from human placenta, inserted into the E. coli replicating plasmid pBR322, and sequenced by Fiddes and Goodman (40,43). These clones (pBR322-c-alpha-hCG and pBR322-c-beta-hCG) were obtained from J. Fiddes (California Biotechnology, Mountainview CA) and the cDNA inserts were transferred into the E. coli replicating plasmid/eukaryotic expression vector pSVL (Pharmacia) to create pSVL-hCG-alpha and pSVL-hCG-beta.

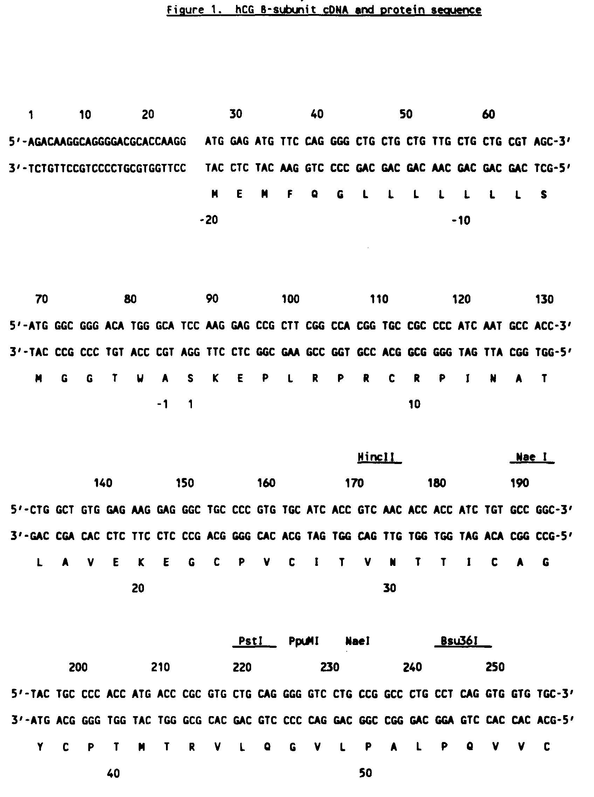

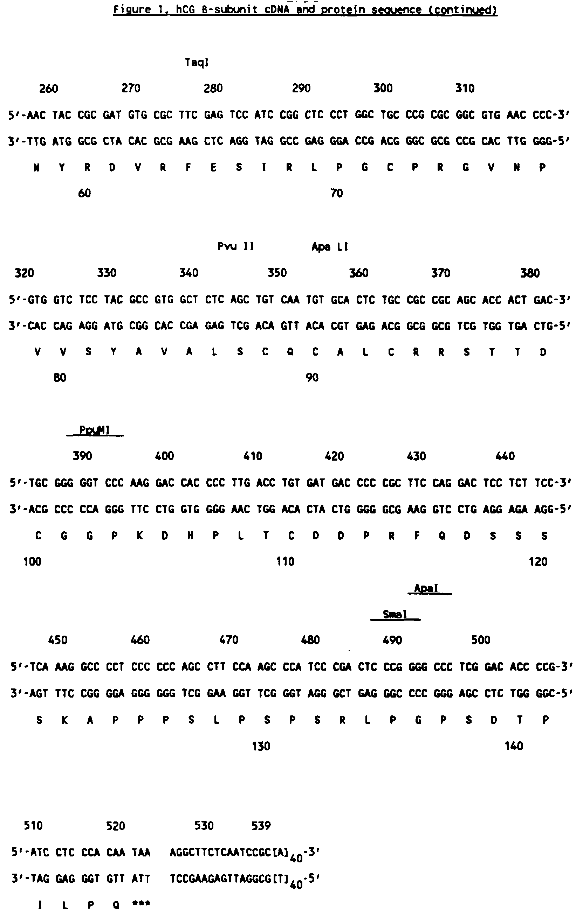

FIGURE 1 depicts the nucleotide sequence of the hCG beta-subunit cDNA, which includes the coding sequence for the mature protein and signal sequence, as well as adjoining 5' and 3' non-translated regions and a poly(A)/poly(T) region corresponding to the poly(A) tail of the mRNA. The hCG beta-subunit amino acid sequence encoded by the cDNA is depicted using the standard single-letter code (see legend for TABLE I) under the nucleotide sequence. The numbering scheme is the same as that used by Fiddes and Goodman (40). Several restriction sites are shown above the nucleotide sequence. Sites unique in pSVL-hCG-beta or pKBM-hCG-beta (see EXAMPLE 2) are underlined.

Ten micrograms (ug) of plasmid pBR322-c-beta-hCG was digested with the restriction enzyme HindIII and the 3'-recessed ends of the cut DNA were filled in with T4 DNA polymerase. The DNA fragment containing the entire 579 base pair (bp) hCG-beta cDNA was purified by electrophoresis in a 1% low melting temperature agarose (Sea Plaque[TM]-FMC, Inc.)/ethidium bromide gel as described elsewhere (42). The DNA fragment containing the hCG-beta cDNA was visualized on a UV light box and recovered by isolating a gel slice containing the DNA with a razor blade. Ten micrograms of plasmid pSVL were cut with the restriction enzyme SmaI and the 5'-phosphate groups were removed from the ends of the cut DNA by treatment with calf intestine alkaline phosphatase. The linearized vector DNA (4889 bp) was purified by electrophoresis and isolated as described above. The gel slices containing the hCG-beta cDNA and linearized pSVL vector DNA were melted by heating at 65° C. for 10 minutes, after which 8 microliters of the hCG-beta cDNA were combined with 2 microliters of pSVL and ligated overnight at 15° C. with 400 units T4 DNA ligase (New England Biolabs) in a volume of 20 microliters. The ligation mix was the heated at 65° C. for 10 minutes, diluted with 180 microliters of CaCl₂/PIPES buffer (60 mM CaCl₂, 10 mM piperazine-N-N'-bis[2-ethanesulfonic acid], pH 7), and 30 microliters of the diluted reaction was used to transform E. coli DH5-alpha cells. Plasmid DNA from ampicillin-resistant clones were screened by co-digestion with the restriction enzymes PstI and XbaI, which produces a DNA fragment of approximately 233 bp if the cDNA is inserted in the correct orientation (i.e., proper orientation for transcription of sense mRNA).

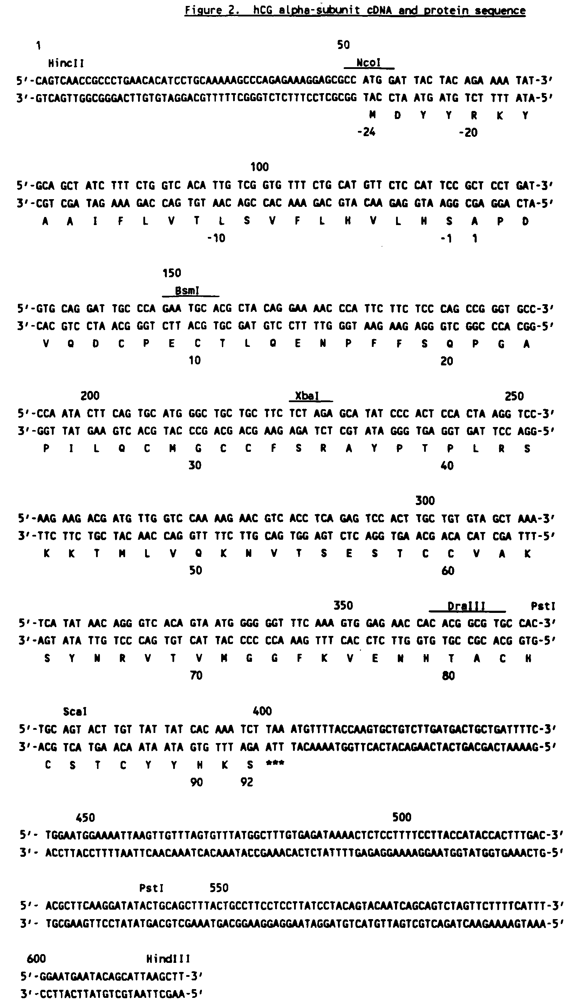

FIGURE 2 depicts the nucleotide sequence of the hCG alpha-subunit cDNA, which includes the coding sequence for the mature protein and signal sequence, as well as adjoining 5' and 3' non-translated regions. The hCG alpha-subunit amino acid sequence encoded by the cDNA is depicted using the standard single-letter code under the nucleotide sequence. The numbering scheme is the same as that used by Fiddes and Goodman (43). Several restriction sites are shown above the nucleotide sequence. Sites unique in pSVL-hCG-alpha or pKBM-hCG-alpha (see EXAMPLE 2) are underlined. The cDNA for the hCG alpha-subunit (43) was placed into pSVL in the same manner as described for hCG-beta, except that screening for pSVL-hCG-alpha was performed by digestion with XbaI alone and selection of clones exhibiting an approximately 400 bp fragment.

These vectors were used to express hCG subunits together or separately in COS cells (44) and as parent constructs for developing plasmids to express and/or modify glycoprotein hormone subunits. In addition, many of the modifications to the cDNA sequences were made directly in pSVL-hCG-beta and pSVL-hCG-alpha. Other plasmid vectors used in the course of this work are pTZ19R (46), pUCX2 (described below), pBMT2X (47) and pCM (described below).

### EXAMPLE 2

### Construction of pKBMt, pKBM, pKBM-hCG-alpha, pKBM-hCG-beta, pUCX2

The E. coli replicating plasmid pKBM is a derivative of pUC18 (45) which has been modified to contain the following polylinker, (starting with the HindIII site of pUC18), HindIII, XhoI, XbaI, SacI (SstI), BamHI, BssHII, SalI, SacII (SstII), and EcoRI. This vector was developed to facilitate cassette mutagenesis of glycoprotein hormones. Unlike pSVL, pKBM and pKBMt lack cleavage sites for the restriction enzymes PstI, PpuMI, NaeI and BsmI among others. The absence of these sites in pKBM facilitated cassette mutagenesis using these sites in hCG alpha and beta-subunit cDNAs.

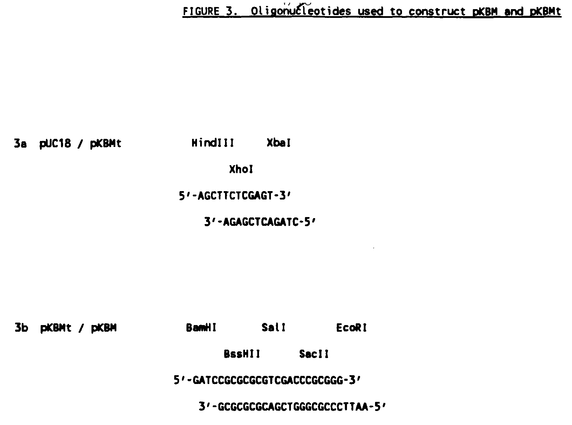

FIGURE 3 depicts the oligodeoxynucleotide cassettes used in the construction of pKBMt (3a) and pKBM (3b). Restriction endonuclease cleavage sites encoded by each cassette are indicated.

Two micrograms of pUC18 were digested with the restriction endonucleases HindIII and XbaI and purified by electrophoresis in low melting point agarose as described in EXAMPLE 1. The two synthetic DNA oligonucleotides depicted in FIGURE 3a were synthesized using an Applied Biosystems 380B DNA Synthesizer and the phosphoramidite method (51), purified by polyacrylamide gel electrophoresis (as described in Applied Biosystems User Bulletin No. 13, April 1, 1987), and quantified by spectrophotometry (absorbance at 260 nm). The oligonucleotides were then annealed by combining 1 microgram of each oligonucleotide in 100 mM KCl, 10 mM Tris pH 8.0, 1 mM EDTA (total volume=200 microliters) heating at 100° C. for five minutes and cooling for 30 minutes at room temperature. Approximately 30 nanograms of HindIII/XbaI cut pUC18 was combined with 100 nanograms of the annealed oligonucleotides and ligated overnight at 15° C. with 1 unit T4 DNA ligase (Bethesda Research Laboratories) in a 20 microliter volume. The ligation reaction was heated at 65° C. for 10 minutes and diluted with 180 microliters of CaCl₂/PIPES. Thirty microliters of the diluted ligation reaction was used to transform E. coli K-12 strain JM83 (87) which were then plated with 50 microliters of a 2% 5-bromo,4-chloro,2-indolyl-beta-D-galactoside (X-gal) DMSO solution. Plasmid DNA from ampicillin-resistant blue colonies were screened with XhoI to verify the presence of the cassette. The resulting plasmid was designated pKBMt. To construct the plasmid pKBM, two additional DNA oligonucleotides were synthesized, annealed to form the cassette depicted in FIGURE 3b, and ligated with EcoRI/BamHI digested pKBMt. Plasmid DNA from ampicillin-resistant blue colonies were screened with BssHII, and plasmids containing the new cassette were designated pKBM. The hCG alpha and beta-subunit cDNAs were subsequently transferred from pSVL into pKBM between the XhoI and BamHI sites to create pKBM-hCG-alpha and pKBM-hCG-beta. The vector pUCX2 is another derivative of pUC18 with an altered polylinker. The polylinker in pUCX2 is (starting from the former HindIII site of pUC18), BssHII, SacII, HindIII, XhoI, and EcoRI.

### EXAMPLE 3

### Construction of pKBM-hCG-beta'

An analog of the hCG beta-subunit cDNA, called hCG-beta', containing two silent mutations which eliminate restriction sites has been the starting construct for many mutants.

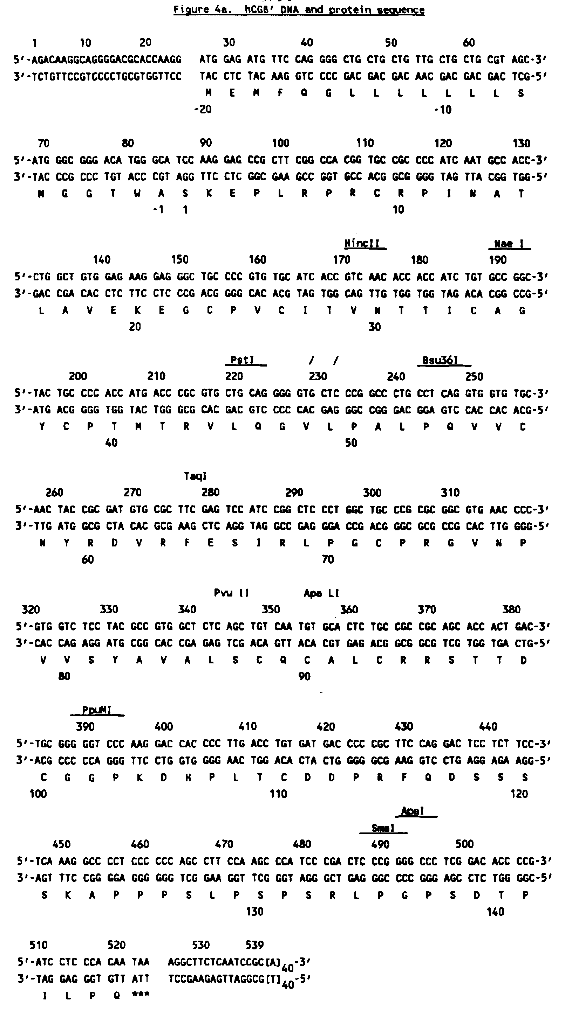

FIGURE 4a depicts the nucleotide sequence of the hCG-beta' DNA. As with the hCG-beta cDNA it includes the coding sequence for the mature protein and signal sequence, adjoining 5' and 3' non-translated regions and a poly(A)/poly(T) region corresponding to the poly(A) tail of the mRNA. The encoded amino acid sequence depicted under the nucleotide sequence is unchanged from that of hCG-beta. The locations of the silent mutations in the DNA sequence are indicated by solid triangles above the base pairs at positions 229 and 232. The numbering scheme is the same as that used by Fiddes and Goodman (40). Several restriction sites are shown above the nucleotide sequence. Sites unique in pSVL-hCG-beta' or pKBM-hCG-beta' are underlined.

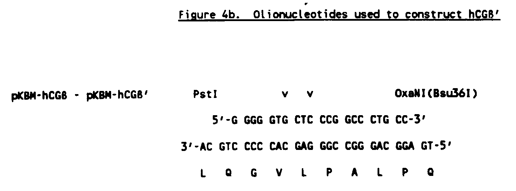

FIGURE 4b depicts the oligonucleotide cassette used to construct pKBM-hCG-beta'. As in FIGURE 4a, the mutated bases are indicated by solid triangles.

Approximately 5 micrograms of pKBM-hCG-beta was digested with the restriction endonucleases PstI and OxaNI (OxaNI is an isoschizomer of Bsu36I and MstII) and the large restriction fragment purified by electrophoresis in low melting point agarose as described above. Two synthetic DNA oligonucleotides (FIGURE 4a) were synthesized, purified and annealed as described in EXAMPLE 2. Two hundred nanograms of PstI/OxaNI cut pKBM-hCG-beta was combined with 80 nanograms of the annealed oligonucleotide cassette, ligated and then used to transform E. coli JM83 as described. Ampicillin-resistant white colonies were picked and plasmid DNA from these clones was screened by digestion with the restriction endonuclease PpuMI, which cuts out an approximately 200 bp fragment from pKBM-hCG-beta, but no fragment from pKBM-hCG-beta'. The hCG-beta DNA in pKBM-hCG-beta' differs from the native hCG-beta cDNA in that it contains unique NaeI and PpuMI restriction sites, facilitating its modification by cassette mutagenesis.

### EXAMPLE 4

### Construction of Analog B9

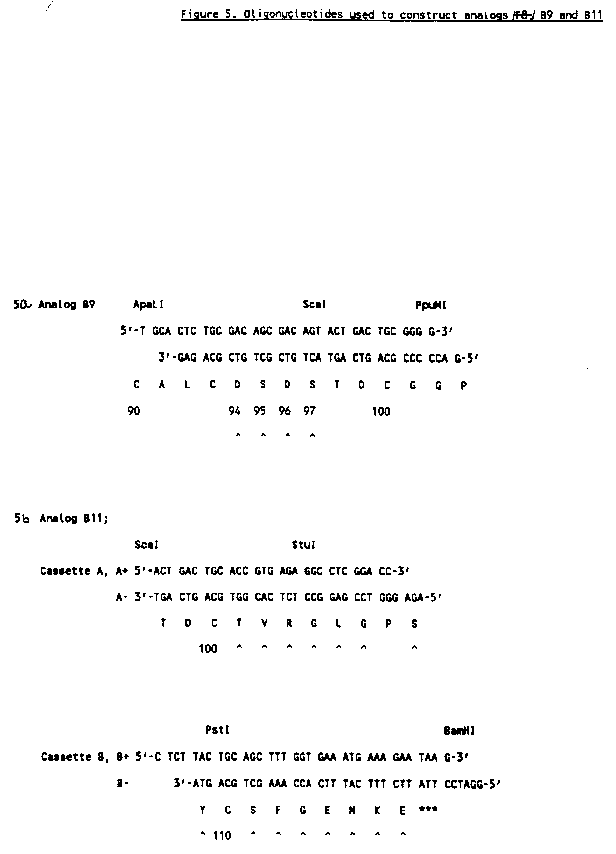

FIGURE 5a depicts the DNA oligonucleotide cassette used to construct analog B9 and the encoded amino acid sequence. The substitution of hFSH beta-subunit residues 88-91 (DSDS) for the corresponding residues of the hCG beta-subunit, i.e., residues 94-97 (RRST) are indicated by solid triangles, and the recognition site for restriction endonuclease ScaI is also noted. Approximately 25 micrograms of plasmid pKBM-hCG-beta' was used in a time-course digestion with 20 units of restriction endonuclease ApaLI in a 100 microliter volume. A portion of the reaction was removed, placed on ice, and adjusted to 20 mM EDTA at time 0 (12 microliters, taken prior to addition of enzyme), and after 10 (12 microliters), 20 (24 microliters), 30 (24 microliters), 45 (12 microliters) and 60 (16 microliters) minutes incubation at 37° C. These were then purified by electrophoresis in low melting point agarose as described in EXAMPLE 1. In this particular time-course optimal linearization of the plasmid was seen after 20 minutes. The bands containing single cut plasmid (approximately 3280 bp) were cut from the gel, transferred to a microfuge tube,and the gel slices melted by incubating at 65° C. for 10 minutes, after which they were diluted with 200 microliters deionized water. Two hundred microliters of phenol (adjusted as described in ref. 41) were then added, the tube vortexed, and then placed in a dry ice/ethanol bath for 15 minutes, after which they were centrifuged at full speed in an Eppendorf microfuge at - 20° C. for 15 minutes. The aqueous fraction was collected and the organic fraction back extracted with an additional 200 microliters deionized water. The aqueous phase from this second extraction was pooled with the aqueous phase from the initial extraction, and then extracted with 400 microliters of chloroform/isoamyl alcohol (24:1 mixture as described in ref. 40), after which the DNA was recovered from the aqueous phase by precipitation with sodium acetate and ethanol. The DNA was resuspended in 14 microliters deionized water, digested with PpuMI, and purified by electrophoresis. The oligonucleotides depicted in FIGURE 5b were synthesized, purified and annealed, and then ligated with the ApaLI/PpuiMI digested pKBM-hCG-beta' DNA. Plasmid DNA from ampicillin-resistant clones of transformed JM83 E. coli was screened for the presence of the insert by digestion with ScaI, which cuts pKBM-hCG-beta' once and cuts pKBM-hCG-beta' containing the insert twice. The new vector is denoted herein as pKBM-B9. The coding sequence for analog B9 was subsequently transferred to pSVL by digesting pKBM-B9 with XhoI and BamHI, gel purifying the approximately 600 bp fragment, and ligating with XhoI/BamHI digested pSVL. Purified preparations of this vector, pSVL B9, also referred to as pSVL-beta-D or pSVL-beta-CF94-97, were sequenced and used to co-transfect COS7 cells with pSVL-hCG-alpha.

### EXAMPLE 5

### Construction of Analog B11

In some instances multiple cassettes were ligated into a vector, as for example the assembly of pSVL-B11. This analog, in which amino acids 94-145 in the mature hCG beta-subunit are replaced with amino acids 88-111 from the mature hFSH beta-subunit, was built using plasmid pSVL-B9 (EXAMPLE 4) and two oligonucleotide cassettes.

FIGURE 5b depicts the DNA oligonucleotide cassettes (A and B comprising oligonucleotides A+ and A-, and B+ and B-, respectively) used to construct analog B11 from B9, and the encoded amino acid sequence. The substitution of hFSH beta-subunit residues 95-111 (TVRGLGPSYCSFGEMKE) for the hCG beta-subunit residues 101-145 (GGPKDHPLTCDDPRFQD...Q, see FIGURE 1 for full sequence) are indicated by the solid triangles, and recognition sites for restriction endonucleases StuI and PstI are noted.

The cassettes were designed so that they could be ligated together (they overlap by 4 complementary, non-palindromic bases) and into the ScaI and BamHI sites of pSVL-B9. The oligonucleotides for the cassettes were synthesized as described for pKBM-hCG-beta' and purified using oligonucleotide purification cartridges from Applied Biosystems. The 5'- ends of oligonucleotides A-and B+ were phosphorylated using T4 polynucleotide kinase to permit ligation of the two cassettes. Oligonucleotides A+ and A-, and B+ and B-, were then combined and annealed as described above. Four micrograms of pSVL-B9 DNA was partially digested with restriction endonuclease ScaI using a time-course, purified by gel electrophoresis, and extracted from the gel as described. The purified ScaI linearized pSVL-B9 DNA was then resuspended in 16 microliters deionized water, digested with restriction endonuclease BamHI, purified by gel electrophoresis, and combined with the two annealed cassettes in a ligation reaction. Ampicillin-resistant clones containing plasmids with the desired insert were isolated by digesting plasmid DNA with PstI, for which there are two recognition sites in pSVL-B9 and three in plasmids with the desired insert, which are denoted pSVL-B11 (also pSVL-beta-DG or pSVL-beta-CF94 117).

### EXAMPLE 6

### Construction of pCM, pCM-hCG-alpha, pCM-hCG-beta

The vector pCM contains the Simian Virus 40 (SV40) origin of replication, early promoter and polyadenylation sequence; the neomycin (aminoglycoside) phosphotransferase gene, ampicillin resistance gene, and bacterial origin of replication (i.e., the EcoRI/BamHI fragment) from pMVBneo (47), the SV40 origin of replication and late promoter, and synthetic polylinker (XhoI-XbaI-SmaI-SstI-BamHI) from pSVL (i.e., the HindIII/BamHI fragment), and the HindIII/EcoRI fragment of Bovine Papilloma Virus (BPV), which contains BpV genes E6, E7, E8, and a portion of E1, the two plasmid maintenance sequences, and an enhancer region (for review see ref. 48). The HindIII/EcoRI fragment from BPV which we placed into pCM has been shown to be capable of controlling replication of plasmids with an SV40 origin of replication permitting the creation of COS cell lines stably transformed with an episomal vector (49).

The plasmid pCM was constructed as follows. Two micrograms of pSVL was digested with restriction endonucleases EcoRI and BamHI and the smaller fragment (approximately 2000 bp) purified by electrophoresis as described in EXAMPLE 1. Two micrograms of plasmid pMVBneo (47), obtained from G.N.Pavlakis (National Cancer Institute, Frederick, MD), was digested with EcoRI and BamHI and the large fragment (approximately 5000 bp) gel purified. These were then ligated and plasmid DNA from ampicillin-resistant clones of DH5-alpha E. coli screened by digestion with HindIII. Clones exhibiting HindIII cut fragments of approximately 1100, 2950 and 2000 bp were positive for the intermediate plasmid, designated pSVLneo. Additional DNA from these clones was then screened by co-digestion with either EcoRI and BamHI (which was expected to yield fragments of approximately 2000 and 5000 bp) or EcoRI, BamHI and HindIII (which was expected to yield fragments of approximately 450, 500, 1100, 2500 and 2500bp). To assemble the final vector, pCM, approximately 5 micrograms of DNA from a clone of pSVLneo was partially digested with 2 units of HindIII in a reaction from which samples were removed, adjusted to 25 mM EDTA (to inactivate the enzyme) and placed on ice at the following time points; 0 (10 microliters), 10 (22 microliters), 20 (22 microliters), 30 (22 microliters) and 45 (22 microliters) minutes after addition of enzyme. These samples were gel purified and the linearized plasmid (approximately 7000 bp) was cut from the gel and transferred to a microfuge tube. The gel slice was melted by incubation at 65° C. for 10 minutes, and 10 microliters was transferred from the microfuge tube to a second tube containing 6 microliters of water, 2 microliters React3 (TM) (BRL) and 20 units of EcoRI. This tube was incubated at 37° C. for an hour, and the approximately 6500 bp EcoRI/HindIII cut band purified by gel electrophoresis. The plasmid pBMT2X (47), obtained from G.N. Paviakis, was digested with HindIII and EcoRI, gel purified, and the 2200 bp fragment corresponding to the 2200 bp fragment of BPV cut from the gel. This fragment was ligated with the 6500 bp EcoRI/HindIII cut fragment of pSVLneo, and plasmid DNA from ampicillin-resistant clones of DM5-alpha E. coli digested with HindIII to identify plasmids with the 2200 insert from pBMT2X (positive clones exhibit bands of approximately 1100, 3000 and 4800 bp). The new plasmid was designated pCM or pSVLneoBPV. The hCG-beta cDNA was inserted between the XhoI and BamHI sites of pCM and plasmid DNA from ampicillin-resistant clones was screened by digestion with PstI or XhoI and BamHI. This new plasmid was designated pCM-hCG-beta. The hCG-alpha cDNA and DNAs encoding hormone analogs were placed into pCM in similar fashion.

The new vector pCM combines attributes of the two principal existing types of eukaryotic expression systems, i.e., transient high level expression and stable expression, into a single system. When transfected into COS cells using procedures described in EXAMPLE 9, pCM-hCG-beta provided rapid, high level expression of recombinant proteins, superior to that obtained with pSVL-hCG-beta. When these transfected COS cells are incubated with medium containing 500 micrograms/ml of synthetic aminoglycoside G418 (Geneticin-GIBCO) it was possible to establish stably transfected cells which produced recombinant proteins at levels of several hundred nanograms/million cells/day for prolonged periods of time (e.g., >30 cell passages), thereby facilitating the cloning and characterization of gene products. These cells were also successfully frozen, stored in liquid nitrogen for several months, and then recultured without loss of the ability to secrete hCG beta-subunit.

It was also possible to recover the DNA vector from the transformed COS cells and reclone it in bacteria. Transformed cells were scraped from the surface of a culture dish and transferred to a 15 ml tube. The cells were then washed twice with 5 ml cold phosphate buffered saline, resuspended in 400 microliters of 0.6% sodium-dodecyl sulfate (SDS)/10 mM EDTA, transferred to a microfuge tube, and incubated for 20 minutes at room temperature. This mixture was then adjusted to 1M NaCl (by addition of 100 microliters of 5M NaCl) and incubated on ice for at least five hours. After this the cellular debris were centrifuged out of solution (full speed in a microfuge for 5 minutes) and the resulting supernatant containing the plasmid was extracted with phenol and precipitated with ethanol and 10 micrograms of mussel glycogen. The isolated DNA was resuspended in 20 microliters of water. One microliter of this was used to transform DH5-alpha E. coli, yielding 63 ampicillin-resistant colonies (we found that these bacteria can also be selected with G418 due to the presence of the neomycin phosphotransferase gene in the vector, even though it is downstream of the SV40 early promoter). Plasmid DNA was prepared from 24 of these clones and digested with XhoI and BamHI. Twenty three of the recovered plasmids were found to contain a fragment of approximately 600 bp corresponding to the hCG-beta cDNA, whereas one clone did not grow in liquid culture. None of the plasmids gave any evidence of rearrangement. This strategy and procedure, previously used with transiently transfected COS cells, is now extended to stable transformed cells containing a hybrid BPV-SV40 vector. Vectors of this type could conceivably be used in any cell type supporting an SV40 origin of replication.

Other related viral origins (e.g., polyoma virus) may work in this system as well. Other promoters, enhancers, selectable markers could also be used, singly or in combination. Vectors such as pCM can be used for cloning genes, characterization of gene products, modification of cells, and any other application involving selection of particular transfected cells from a pool of cells.

### EXAMPLE 7

### Construction of pBMT2X-hCG-alpha and pBMT2X-hCG-beta,

The vector pBMT2X permits stable expression of recombinant proteins in C127 cells (47). hCG subunits and analogs were sub-cloned into the unique XhoI site of pBMT2X (provided by Dr.G.N. Pavlakis, see above, EXAMPLE 6). Approximately 10 micrograms of plasmids pKBM-hCG and pKBM-hCG-beta were digested with restriction endonucleases XhoI and SalI and the approximately 600 bp fragments containing the alpha-subunit and beta-subunit cDNAs were isolated as described in EXAMPLE 1. Two micrograms of plasmid pBMT2X was digested with XhoI, the 5' terminal phosphates removed from the DNA with calf intestinal alkaline phosphatase, and the linearized DNA gel purified as described in example 1. The XhoI digested pBMT2X and cDNA fragments were ligated with T4 DNA ligase and used to transform HB101 E. coli as described for DH5-alpha E. coli in EXAMPLE 1. Plasmid DNA from ampicillin-resistant clones was digested with BssHII, which linearized clones containing the cDNA inserts from pKBM. The orientation of the inserts was determined by digestion of the plasmid DNA with XhoI and SstII, with inserts in the proper orientation exhibiting a fragment of approximately 1400 bp. These new plasmids were designated pBMT2X-hCG-alpha, pBMT2X-hCG-beta. Plasmid preparations of pBMT2X-hCG-alpha and pBMT2X-hCG-beta were subsequently used to transform C127 cells as previously described (50, see also EXAMPLE 11) and cells producing either hCG dimer, or alpha or beta were sub-cloned in the presence of 20-50 uM cadmium chloride (CdCl₂). Production of protein by these cells was on the order of 5 micrograms hCG dimer/million cells/24 hours.

### EXAMPLE 8

### METHODS OF MUTAGENESIS

### Oligonucleotide Cassette Mutagenesis

Many constructs have been prepared by DNA oligonucleotide cassette mutagenesis similar to the manner in which pKBMhCG-beta', pKBM-B9 or pSVL-B18 were built.

### Restriction Fragment Mutagenesis

Vectors containing native cDNAs, genes or analogs thereof with restriction sites in common can be used to create new analogs by replacing restriction fragments.

### EXAMPLE 9

### Introduction of Genetic Vectors into Mammalian Cells

Both calcium phosphate and DEAE-dextran methods have been used to transfect COS (COS-1 or COS-7) cells with vectors containing glycoprotein hormone subunits or analogs. The calcium phosphate technique was a modification of a published protocol(50). COS cells to be transfected were plated the day before on 6-cm or 10-cm diameter tissue culture plates at a density of 10,000 cells per square centimeter. Plasmid DNA was purified in an ultracentrifuge over a CsCl gradient and sterilized by precipitation with ethanol. The quantity of DNA used in the transfection procedure varied from 1 to 20 micrograms per plate of cells. The final volume of the DNA solution was either 400 microliters (6-cm plate) or 1 ml (10-cm plate). Salmon sperm DNA was sometimes used as a carrier. After resuspending the DNA in sterile water, an appropriate volume of 2 M CaCl₂ was added to yield a final concentration of 125 mM of CaCl₂ in 200 microliters (for each 6-cm plate) or 500 microliters (for each 10-cm plate). An equal volume of 2X BES-buffered saline (50 mM of N-,N-Bis[2-hydroxyethyl]-2-aminoethanesulfonic acid, 280 mM NaCl, 1.5 mM Na₂HPO₄; adjusted to pH 6.95) was then added to the DNA/CaCl₂ solution. This mixture was vortexed, incubated at room temperature for 10-20 minutes and then added drop-wise to the plates of cells. The cells and DNA were incubated overnight (18-30 hours in a 37° C. incubator under 2.5-6% carbon dioxide. The medium was removed, the cells washed with 1X PBS and then the cells were cultured in complete medium. After 12-24 hours this media was removed, the cells were washed twice with 10 ml PBS, and serum-free DMEM was added. The media was harvested 70-80 hours later for recovery of secreted protein.

Transfections using DEAE-dextran were done according to a published protocol (86). In this procedure, one million cells were plated on 100 mm plates the day before the transfection. Plasmid DNA was prepared and sterilized as described above, then resuspended in sterile water, adjusted to 1x PBS concentration and 200 micrograms/ml DEAE-dextran in 1.2 ml. Media was aspirated from the plates of cells, and the plates were washed twice with PBS. The DNA/DEAE-dextran solution was then added to the plates, which were placed in a 37° C. incubator for 40 minutes. After this time, 10 ml DMEM made 100 uM with chloroquine was added to each plate. The plates were incubated for 3-4 hours, after which the media was replaced with medium containing 10% DMSO. Following 2-3 minutes at 37° C. the medium was aspirated, the plates were washed with PBS, covered with 10 ml DMEM containing 10% FCS or serum substitute (Calf Supreme or Calf Serum, Supplemented from GIBCO) and placed back in the incubator. After 12-24 hours the medium was removed, the plates washed twice with 10 ml PBS, and then covered with 6 ml serum-free DMEM. The medium was harvested 70-80 hours later for recovery of secreted protein.

Stable transfections of COS, C127 or CHO cells were performed using the calcium phosphate procedure described above. Stable clones of C127 cells transfected with pBMT2X-based plasmids were selected by resistance to CdCl₂ (10-80 mM) starting two days after transfection. Cells transfected with plasmids containing the gene for neomycin (aminoglycoside)phosphotransferase (e.g, pCM-based plasmids) were selected with the synthetic aminoglycoside G418 (Sigma) by adding 500 micrograms/ml media G418/ml tissue culture medium (COS) or 1000 micrograms G418/ml medium(CHO) starting 1-4 days after transfection. After this the cells were routinely maintained under selection pressure.

### EXAMPLE 10

### Sandwich Assays

Immunological properties of the hormones and analogs were determined using monoclonal sandwich assays essentially as previously described by Moyle et al. (5). These assays were used to determine whether the analogs folded similar to the native molecule and formed alpha, beta-heterodimers which were secreted into the culture medium.

TABLE III summarizes results of the immunological characterization of hCG/hFSH chimeras. All assays employed B105 as "capture" antibody, except for assays of hFSH, which used B601, B602, A113 or E501 (see below). The various labeled detection antibodies are indicated in the TABLE. The specificity of these antibodies has been described previously (5-7, 52, 53). In these assays, 1 microgram of capture antibody (e.g., B105) was adsorbed to the surface of individual wells in 96-well microtiter plates by adding 50 microliters of a 0.9% NaCl/20 micrograms/ml antibody solution (made with distilled water) to each well and incubating at 37° C. for 1 hour. Unbound antibody was removed and non-specific sites were blocked with bovine serum albumin (BSA) by filling each well with a 0.1% BSA/0.9% NaCl solution and incubating at 37° C. for 1 hour. Hormone standards or analogs were added to each well (volume adjusted to 50 microliters) incubated for 1 hour at 37° C. to permit the analogs to bind to the adsorbed antibody. The wells were washed with distilled water to remove unbound analogs and 50,000 counts per minute (cpm) of labeled "detection" antibody in BSA/saline (50 microliters volume) was added to the well and incubated at 37° C. for 1 hour. Next, the unbound antibody was removed by aspiration, the plates washed with deionized water, and the individual wells cut out and counted in a gamma counter. Detection antibodies were radioiodinated by the iodogen method as previously described by Moyle et al. (54).

TABLE III illustrates the ability of monoclonal antibody sandwiches which detect the alpha, beta-heterodimer (B105 capture, A113 detection), hCG beta-subunit or hCG (B105 capture, B101 detection), and analogs with beta-subunit epitopes from both hCG (B105) and hFSH (B601). The ability of the radioiodinated detection antibody to bind to the captured hormone or analog relative to hCG (except for B601) is indicated in TABLE III. Note that the data in TABLE III are expressed as either ++ (cpm bound = 50-120% of those bound to a urinary hCG standard), + (cpm bound = 10-49% of those bound to the standard), or - (<10%binding), and nt indicates not tested. For B601, a monoclonal antibody specific for hFSH, binding to the various analogs is described relative to their reactivity in an hFSH assay, i.e., E501/B601 sandwich assay. In this assay, employing E501 capture, B601 was found to bind chimeras containing hFSH residues 33-52 indistinguishably from recombinant hFSH. The immunologic data demonstrates that the analogs fold and bind to the alpha-subunit. It also demonstrates that structural features of one hormone (e.g., the B601 epitope in FSH) can be transferred to another hormone, even in regions of very low homology.

### EXAMPLE 11

### Assay of Receptor Binding

Two types of assays were used to characterize receptor binding. One is a competitive assay while the other is a sandwich or BioIRMA assay. Both have been described previously by Moyle et al. (20, 54).

Membrane bound LH receptors were obtained from rat corpora lutea super-ovulated with pregnant mares serum gonadotropin and hCG (54). Membrane bound FSH receptors were obtained from bovine calf testes (11). In the BioIRMA, hormones or analogs were incubated with a preparation of membrane bound receptors. Hormone which became bound to the receptors was quantified using radiolabeled B105 monoclonal antibody (6) which has high affinity for an epitope on the hCG beta-subunit that remains exposed after the hormone has complexed with receptors.

Further characterization of the analog's receptor binding properties was determined by radioligand receptor assays as described by Moyle et al. (20). This assay is a competitive displacement assay in which the analogs compete for receptor binding with radioiodinated hCG and hFSH.

FIGURE 7 illustrates tha abilities of hCG, hFSH and analogs to inhibit the binding of ¹²⁵I-hCG to LH receptors. Their ability to inhibit ¹²⁵I-hCG binding correlates to their ability to bind LH receptors. Data from each analog are marked using the nomenclature in TABLE II. Data from analogs with hCG-like activity are plotted with filled boxes, rhCG (―), B15 (-·-), B27 (^{····}); data from analogs with hFSH-like activity are plotted with open boxes, rhFSH (-), B11 (--), B17 (---), B18 (-·-); and data from analogs that behave differently from both hCG and hFSH are plotted with open triangles, B9 (--) and B16 (---).

FIGURE 8 illustrates the abilities of hCG, hFSH and analogs to inhibit binding of ¹²⁵I-hFSH to FSH receptors. Their ability to inhibit ¹²⁵I-hFSH binding correlates to their ability to bind FSH receptors. Representation of hormones and analogs is the same as for LH receptor binding in FIGURE 7.

### EXAMPLE 12

### Assay of Hormone Responses

The ability of hCG, FSH, and the analogs to stimulate steroidogenesis was monitored by radioimmunoassay of testosterone production by rat Leydig cells and estradiol synthesis by rat granulosa cells using procedures similar to those described previously (20, 55).

Rat Leydig cells were isolated and prepared for incubation similar to the procedure described previously (57). Briefly, this procedure consisted of digesting decapsulated rat testes from 22- to 40-day-old Sprague-Dawley rats (Charles River,Wilmington, Mass.), in Krebs Ringer buffer (58) containing 1 mg HEPES in place of bicarbonate and 1 mg collagenase/ml (Worthington, Freehold, N.J.) until the tubules dissociated from one another (10 to 20 minutes). After the addition of 25 ml of enzyme-free buffer to the digestion flasks, the tubules were allowed to sediment and the interstitial cells were decanted into centrifuge tubes. Following centrifugation at 70 x g for 10 minutes the cells were resuspended in the buffer containing 1 mg of bovine serum albumin and counted in a hemocytometer. The routine steroidogenesis assay consisted of adding 50 microliters of the cell suspension containing approximately 350,000 nucleated cells to 50 microliters of hormone or analog in a plastic tube (12 x 75 mm), and measuring the testosterone produced after a 3-hour incubation at 37° C.

Testosterone was measured with a radioimmunoassay procedure utilizing antisera obtained from Cambridge Medical Diagnostics (Billerica, MA). In this procedure, an aliquot of unextracted incubation medium (50 microliters) containing approximately 175,000 cells was added to 0.2 pmol of ³H-testosterone (80 Ci/mmol, New England Nuclear, Boston, MA) dissolved in 100 microliters of "gelatin/phosphate" buffer. This buffer contained 0.01 M potassium phosphate (pH 7), 0.82% NaCl, 0.1% NaN₃, and 0.2% Ditto brand gelatin. Anti-testosterone serum sufficient to bind 0.06 pmol of testosterone was added in 50 microliters of gelatin/phosphate buffer and incubated 1 hour at 37° C. Following an additional 15 minutes in an ice slurry, 400 microliters of dextran/charcoal solution [prepared by adding 1.25 g of powdered charcoal (Sigma, St.Louis, Mo.) to 0.125 g of dextran T-70 (Sigma) and 400 ml of gelatin phosphate buffer] were added to separate the bound and free testosterone. After 15 minutes at 4° C., the mixture was centrifuged at 800 x g for 10 minutes and 400 microliters of the supernatant were added to 4 ml of scintillation fluid (Liquiscent, National Diagnostics). The assay measured 0.02-10 pmol of nonlabeled testosterone.

Granulosa cells were isolated from ovaries of rats (21-27 days old) which had been implanted with 3 diethylstilbestrol(DES) "pellets" made by packing solid DES into 1 cm long sialastic tubings. Estrogen treatment has been shown to induce granulosa cell hyperplasia (60) without altering the number of FSH receptors per cell (61). Granulosa cells were cultured using a minor modification (59) of the procedure described by Ericksonand Hsueh (62). Cells were suspended in McCoys 5a medium (GIBCO, Grand Island, NY) and either 100 microliters containing 40,000-50,000 cells or one milliliter containing 400,000-800,000 cells was added to each well of a standard 96- or 24-well culture plate. The medium also contained penicillin, streptomycin, and 100 nM androstenedione. Since granulosa cells lack the ability to convert C-21 steroids to C-19 steroids (62), the androstenedione was needed as a substrate for estradiol synthesis. In addition, androstenedione serves as a stimulus for progesterone formation (63, 64) although it is not needed for this function. The medium was not changed during the culture interval. Estradiol and progesterone were measured directly from 0.02-0.2 ml culture fluid by RIA using highly specific antisera (65).

FIGURE 9 is a dose response curve of LH receptor mediated steroidogenic activity of hCG/hFSH chimeric beta-subunits co-expressed in COS7 cells with human alpha-subunit, compared with hCG and hFSH. Data from each analog are marked using the nomenclature from TABLE II. Data from analogs with hCG-like activity are plotted with open boxes, rhCG (―), B15 (-·-), B27 (····); data for rhFSH (―) are plotted with filled boxes; data from analogs with slightly diminished hCG-like activity are plotted with open triangles, B9 (--) and B16 (---); and data from analogs with moderate hCG-like activity are plotted with filled triangles, B11 (--), B17 (---) and B18 (-·-). The steroidogenic potency of the analogs in this assay is strongly related to their ability their receptor binding activity (see FIGURE 7).

FIGURE 10 is a dose response curve of FSH receptor mediated steroidogenic activity of hCG/hFSH chimeric beta-subunits co-expressed in COS7 cells with human alpha-subunit, compared with hCG and hFSH. Data from each analog are marked using the nomenclature from TABLE II. Data from analogs with hFSH-like activity are plotted with filled triangles, B11 (--), B17 (---) and B18 (-·-); data from hFSH (―) are plotted with filled boxes; and data from rhCG (―) are plotted with open boxes. The steroidogenic potency of these analogs in this assay is strongly related to their FSH receptor binding activity (see FIGURE 8).

All references cited hereinabove and hereinafter, are incorporated by reference.

### REFERENCES

1. Pierce, J.G. and Parsons. T.F., Ann. Rev. Biochem. 50, 465 (1981).
2. Ryan, R.J. et al., Recent Progress Hormone Res. 43, 383 (1987).
3. Sairam, M.R., Arch. Biochem. Biophys. 204, 199 (1980).
4. Keutmann, H.T. et al., Biochem. 22, 3067 (1983).
5. Moyle, W.R. et al., Proc. Natl. Acad. Sci. (USA) 79, 2245 (1982).
6. Ehrlich, P.H. et al., Am. J. Immunol. Microbiol. 8, 48 (1985).
7. Schwartz, S. et al., Endocrinol. 118, 189 (1986).
8. Berger, P. et al., Endocrinol. 123, 2351 (1988).
9. Charlesworth, M.C. et al., J. Biol. Chem. 262, 13409 (1987).
10. Keutmann, H.T. et al., Proc. Natl. Acad. Sci. (USA), 84, 2038 (1987).
11. Sluss, P.M. et al., Biochem. 25, 2644 (1986).
12. Schneyer, A.L. et al., Biochem. 27, 666 (1988).
13. Strickland, T.W. and Puett. D., Int. J. Peptide Protein Res. 21, 374 (1983).
14. Armstrong. E.G. et al., Biochemical Actions of Hormones (G. Litwack, Ed.), 13, 91 (1986).
15. Campbell, R.K. et al., Excerpta Medica Int'l. Congr., Series 798, pp. 123-132 (1988).
16. Strickland, T.W. and Puett, D., Endocrinol. 111, 95 (1982).
17. Kusuda, S. and Dufau, M.L., J. Biol. Chem. 261, 16161 (1986).
18. Hwang, J. and Menon, K.M.J., Proc. Nat'l. Acad. Sci.(USA) 81, 4667 (1984).
19. Ji, I. and Ji, T.H., Proc. Nat'l. Acad. Sci. (USA) 78, 5464 (1981).
20. Moyle, W.R. et al., J. Biol. Chem. 250, 9163 (1975).
21. Goverman, J.M. et al., J. Biol. Chem. 257, 15059 (1982).
22. Matzuk, M.M. et al., 1988 Endocrine Soc. Meeting, New Orleans, Abst. #423 (1988).
22a. Matzuk. M.M. et al., J. Cell Biol. 109, 1429-1438 (October 1989), The Rockefeller University Press.
23. Calvo, F.O. and Ryan, R.J., Biochem. 24, 1953 (1985).
24. Keutmann, H.T. et al., FEBS Letters 185, 333 (1985).
25. Bewley, T.A. and Sairam, M.R., Int. J. Peptide Protein Res. 26, 612 (1985).
26. Moyle, W.R. et al., J. Biol. Chem. 262, 16920 (1987).
27. Rebois, R.V. and Fishman, P.H., J. Biol. Chem. 259, 8087 (1984).
28. Manjunath, P. and Sairam. M.R., J. Biol. Chem. 258, 3553 (1983).
29. Hojo, H. and Ryan, R.J., Endocrinol. 117, 2428 (1985).
30. Reddy, V.B. et al., Proc. Nat'l. Acad. Sci. (USA) 82, 3644 (1985).
31. Lustbader, J. et al., J. Biol. Chem. 262, 14204 (1987).
32. Corless, C.L. et al., J. Cell Biol. 104, 1173 (1987).
33. Corless, C.L. et al., J. Biol. Chem. 262, 14197 (1987).
34. Kaetzel, D.M. et al., Proc. Nat'l. Acad. Sci. (USA) 82, 7280 (1985).
35. Matzuk, M.M. et al., Proc. Nat'l. Acad. Sci. (USA) 84, 6354 (1987).
36. Kaetzel, D.M. and Nilson, J.H., J. Biol. Chem. 263, 6344 (1988).
37. Wang, C., Endocrine Rev. 9, 374 (1988).
38. Jones, W.R. et al., Lancet 1, 1295 (1988).
39. Gallagher, P. et al., J. Cell. Biol. 107, 2059 (1988).
40. Fiddes. J.C. and Goodman. H.M., Nature 286, 684 (1980).
41. Maniatis, T. et al., "Molecular Cloning: A Laboratory Manual", Cold Spring Harbor Laboratory (1982).
42. Struhl, K., Biotechniques 3, 6 (1985).
43. Fiddes, J.C. and Goodman, H.M., Nature 281, 351 (1979).
44. Gluzman, Y., Cell 23, 175 (1981).
45. Yarnisch-Perron, C. et al., Gene 33, 103 (1985).
46. Mead, D.A. et al., Protein Engineering 1, 67 (1986).
47. Pavlakis, C.N. et al., "Gene Transfer Vectors for Mammalian Cells", ed. Miller, J.H. and Calos, M.P., Cold Spring Harbor Laboratory (1987).
48. Stephens, P.E. and Hentschel, C.G., Biochemical J. 248, 1 (1987).
49. Roberts, J.M. and Weintraub. H., Cell 52, 397 (1988).
50. Chen, C.A. and Okayama, H., Mob. Cell. Biol. 7, 2745 (1987).
51. Beaucage, S.L. and Caruthers, M.H., Tetrahedron Letters 22, 1859 (1981).
52. Norman, J. et al., J. Clin. End. Metab. 61, 1031 (1985).
53. Milius, R.P. et al., Proc. Nat'l. Acad. Sci. (USA) 80, 7375 (1983).
54. Moyle, W.R. et al., J. Recentor Res. 8, 419 (1988).
55. Skaf, R.A. et al., Endocrinology 117, 106 (1985).
56. Dufau, M.L. et al., J. Biol. Chem. 248, 6973 (1973).
57. Moyle, W.R. and Ramachandran, J., Endocrinology 93, 127 (1973).
58. Umbreit, W.W. et al., "Manometric Techniques", 4th ed.,page 132, Brugess Publ. Co. (1964).
59. Moyle, W.R. et al., Am. J. Physiol. 235, E218 (1978).
60. Goldenberg, R.L. and Vaitukaitis, J.L., Endocrinology 90, 1492 (1972).
61. Louvet, J.P. and Vaitukaitis, J.L., Endocrinology 99, 758 (1976).
62. Erickson, G.F. and Hsueh, A.J.W., Endocrinology 102, 1275 (1978).
63. Nimrod, A. and Lindner, H.R., Mol. Cell Endocrinology 5, 315 (1976).
64. Lucky, A.W. et al., Endocrinology 100, 128 (1977).
65. Powell, J.E. and Stevens, J.C., Clin. Chem. 19, 210 (1973).
66. Schneyer, A.L. et al., Biochemistry 27, 666 (1988).
67. "Recombinant Hormone Challenges Pig Protein for Superovulating Cows", Newswatch, May 4, 1987.
68. Dinsomore, R.P. et al., J. Am. Vet. Med. Assoc. 195, 327 (1989).
69. Stevens, V.C. et al., Immunology Letters 12, 11 (1986).
70. Sekine, S. et al., Proc. Nat'l. Acad. Sci (USA) 86, 8645 (1989).
71. Harris, D.C. et al., J. Biol. Chem. 264, 6705 (1989).
72. Lustbader, J.W., et al., Biochem. 28, 9239 (1989).
73. Birken, S. et al., Endocrinology 123, 572 (1988).
74. Krichevsky, A. et al., Endocrinology 123, 584 (1988).
75. Manning, M.C. et al., Pharm. Res. 6, 903 (1989).
76. Santa Colmoa, T.A. et.al., Biochem. 29, 1194 (1990).
77. Watkins, P.C. et. al., DNA 6, 205 (1987).
78. Noce, T. et al., J. Molec. Endocrinology 3, 129 (1989).
79. Bousfield, G.R. et al., J. Biol. Chem. 262, 8610 (1987).
80. Talmadge, K., et al., Nature 307, 37 (1984).
81. Maurer, R.A., J. Biol. Chem. 260, 4684 (1985).
82. Hayashizaki. Y., FEBS Lett. 188, 394 (1985).
83. Virgin, J.B., et al., J. Biol. Chem. 260, 7072 (1985).
84. Goodwin, R.G., et al., Nucleic Acids Res. 11, 6873 (1983).
85. Stewart, F., et al., J. Endocrinology 115, 341 (1987).
86. Cullen, B. R., Methods Enzymol. 152, 684 (1987).
87. Messing. J., Recombinant DNA Technical Bulletin, NIH, Publication No. 79-009, 2, N0. 2, 43-48, (1979).

The invention being thus described, it will be obvious that the same may be varied in many ways. Such variations are not to be regarded as a departure from the spirit and scope of the invention and all such modifications are intended to be included within the scope of the following claims.

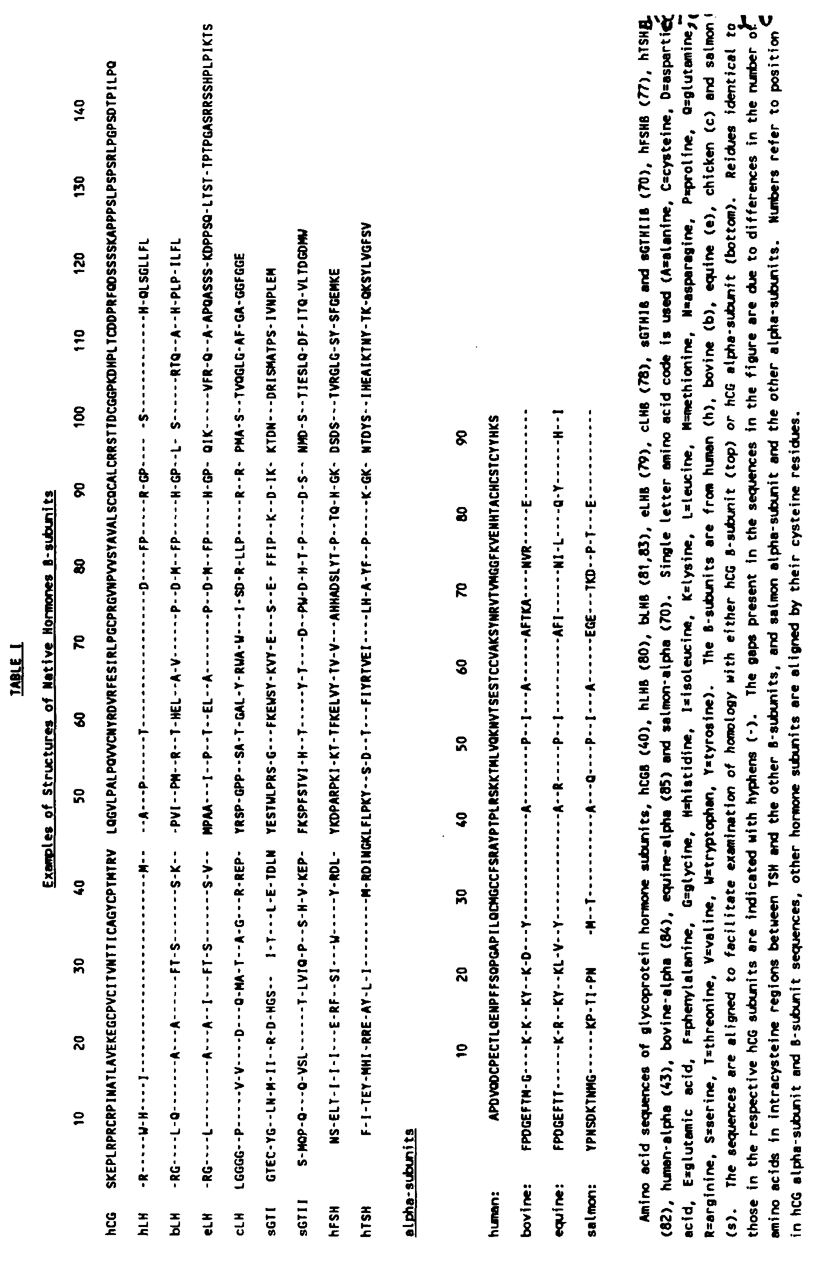

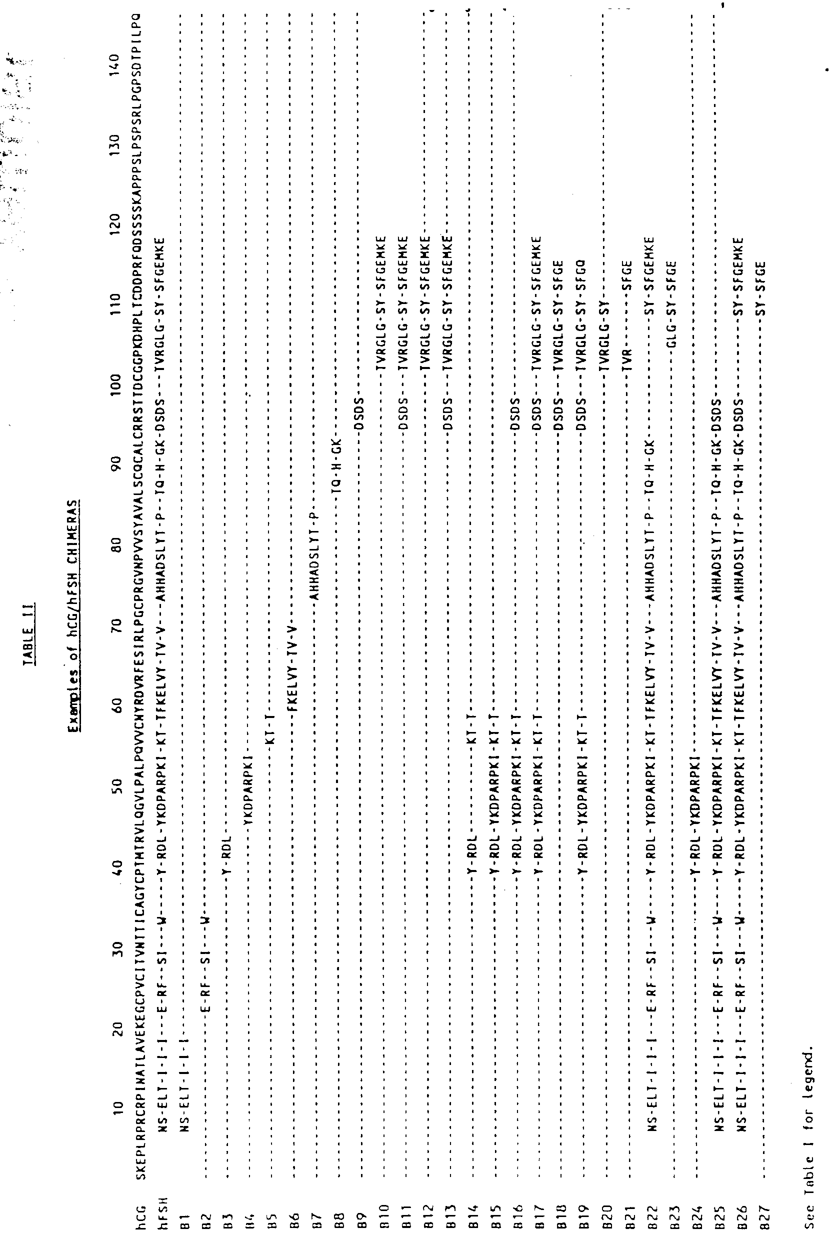

**TABLE III**

| MONOCLONAL ANTIBODY BINDING TO CHIMERIC hCG/hFSH B-SUBUNITS CO-EXPRESSED WITH alpha-SUBUNIT | | | | | |
|---|---|---|---|---|---|
| B-Subunit or Analog | Monoclonal Antibody | | | | |
| | A113 | B101 | B109 | B601 | A109 |
| rhCG | ++ | ++ | ++ | - | ++ |
| B3 | ++ | nt | ++ | nt | + |
| B9 | ++ | ++ | - | - | ++ |
| B11 | ++ | ++ | nt | - | nt |
| B14 | ++ | nt | - | nt | + |
| B15 | ++ | - | - | ++ | - |
| B16 | ++ | - | - | ++ | - |
| B17 | ++ | - | nt | ++ | nt |
| B18 | ++ | ++ | - | - | ++ |
| B28 | ++ | - | - | ++ | - |
| B27 | ++ | ++ | nt | - | nt |
| rhFSH | ++ | - | - | ++ | nt |
| Data are presented as antibody binding to chimeric proteins relative to binding to hCG or hFSH standard; ++ = 50%-110%, + = 10%-50%, - = less than 10%, nt = not tested | | | | | |

## Claims

1. An alpha, beta-heterodimeric polypeptide having greater binding affinity to follicle stimulating hormone (FSH) receptors than chorionic gonadotropins (CG) comprising a glycoprotein hormone alpha-subunit polypeptide and a non-naturally occurring beta-subunit polypeptide, wherein the beta-subunit polypeptide is a chimera consisting of the beta-subunit of human chorionic gonadotropin (hCG) whereby in the C-terminal region the amino acid sequence of residues 101-109 of the beta-subunit of hCG is replaced by the amino acid sequence of residues 95-103 of the beta-subunit of hFSH.

2. An alpha, beta-heterodimeric polypeptide having greater binding affinity to follicle stimulating hormone (FSH) receptors than chorionic gonadotropins (CG) comprising a glycoprotein hormone alpha-subunit polypeptide and a non-naturally occurring beta-subunit polypeptide, wherein the beta-subunit polypeptide is a chimera consisting of the beta-subunit of human chorionic gonadotropin (hCG) whereby in the C-terminal region the amino acid sequence of residues 94-109 of the beta-subunit of hCG is replaced by the amino acid sequence of residues 88-103 of the beta-subunit of hFSH.

3. An alpha, beta-heterodimeric polypeptide having greater binding affinity to follicle stimulating hormone (FSH) receptors than chorionic gonadotropins (CG) comprising a glycoprotein hormone alpha-subunit polypeptide and a non-naturally occurring beta-subunit polypeptide, wherein the beta-subunit polypeptide is a chimera consisting of the beta-subunit of human chorionic gonadotropin (hCG) whereby in the C-terminal region the amino acid sequence of residues 101-106 of the beta-subunit of hCG is replaced by the amino acid sequence of residues 95-100 of the beta-subunit of hFSH.

## Patentansprüche

1. Alpha-, beta-heterodimeres Polypeptid, das eine größere Bindungsaffinität zu Rezeptoren des follikelstimulierenden Hormons (FSH) als Choriongonadotropine (CG) aufweist, umfassend ein Glycoproteinhormon-Alpha-Untereinheit-Polypeptid und ein nicht-natürlich auftretendes Beta-Untereinheit-Polypeptid, wobei das Beta-Untereinheit-Polypeptid eine Chimere ist, die aus der Beta-Untereinheit des menschlichen Choriongonadotropins (hCG) besteht, wobei in der C-terminalen Region die Aminosäuresequenz der Reste 101 bis 109 der Beta-Untereinheit von hCG durch die Aminosäuresequenz der Reste 95 bis 103 der Beta-Untereinheit von hFSH ausgetauscht ist.

2. Alpha-, beta-heterodimeres Polypeptid, das eine größere Bindungsaffinität zu Rezeptoren des follikelstimulierenden Hormons (FSH) als Choriongonadotropine (CG) aufweist, umfassend ein Glycoproteinhormon-Alpha-Untereinheit-Polypeptid und ein nicht-natürlich auftretendes Beta-Untereinheit-Polypeptid, wobei das Beta-Untereinheit-Polypeptid eine Chimere ist, die aus der Beta-Untereinheit des menschlichen Choriongonadotropins (hCG) besteht, wobei in der C-terminalen Region die Aminosäuresequenz der Reste 94 bis 109 der Beta-Untereinheit von hCG durch die Aminosäuresequenz der Reste 88 bis 103 der Beta-Untereinheit von hFSH ausgetauscht ist.

3. Alpha-, beta-heterodimeres Polypeptid, das eine größere Bindungsaffinität zu Rezeptoren des follikelstimulierenden Hormons (FSH) als Choriongonadotropine (CG) aufweist, umfassend ein Glycoproteinhormon-Alpha-Untereinheit-Polypeptid und ein nicht-natürlich auftretendes Beta-Untereinheit-Polypeptid, wobei das Beta-Untereinheit-Polypeptid eine Chimere ist, die aus der Beta-Untereinheit des menschlichen Choriongonadotropins (hCG) besteht, wobei in der C-terminalen Region die Aminosäuresequenz der Reste 101 bis 106 der Beta-Untereinheit von hCG durch die Aminosäuresequenz der Reste 95 bis 100 der Beta-Untereinheit von hFSH ausgetauscht ist.

## Revendications

1. Polypeptide α,β-hétérodimère ayant une plus grande affinité de liaison pour les récepteurs de la hormone folliculostimulante (FSH) que les gonadotrophines chorioniques (CG), comprenant un polypeptide de sous-unité α d'une hormone glycoprotéinique et un polypeptide de sous-unité β n'existant pas dans la nature, le polypeptide de sous-unité β étant une chimère constituée de la sous-unité β de la gonadotrophine chorionique humaine (hCG) dans laquelle, dans la région C-terminale, la séquence d'aminoacides constituée des résidus 101-109 de la sous-unité β de la hCG est remplacée par la séquence d'aminoacides constituée par les résidus 95-103 de la sous-unité β de la hFSH.

2. Polypeptide α,β -hétérodimère ayant une plus grande affinité de liaison pour les récepteurs de la hormone folliculostimulante (FSH) que les gonadotrophines chorioniques (CG), comprenant un polypeptide de sous-unité α d'une hormone glycoprotéinique et un polypeptide de sous-unité β n'existant pas dans la nature, le polypeptide de sous-unité β étant une chimère constituée de la sous-unité β de la gonadotrophine chorionique humaine (hCG) dans laquelle, dans la région C-terminale, la séquence d'aminoacides constituée des résidus 94-109 de la sous-unité β de la hCG est remplacée par la séquence d'aminoacides constituée par les résidus 88-103 de la sous-unité β de la hFSH.

3. Polypeptide α,β-hétérodimère ayant une plus grande affinité de liaison pour les récepteurs de la hormone folliculostimulante (FSH) que les gonadotrophines chorioniques (CG), comprenant un polypeptide de sous-unité α d'une hormone glycoprotéinique et un polypeptide de sous-unité β n'existant pas dans la nature, le polypeptide de sous-unité β étant une chimère constituée de la sous-unité β de la gonadotrophine chorionique humaine (hCG) dans laquelle, dans la région C-terminale, la séquence d'aminoacides constituée des résidus 101-106 de la sous-unité β de la hCG est remplacée par la séquence d'aminoacides constituée par les résidus 95-100 de la sous-unité β de la hFSH.
